# EUROPEAN PATENT SPECIFICATION

(11) **EP 1 189 611 B1**
(45) Date of publication and mention of the grant of the patent: **03.05.2006**
(21) Application number: 00942571.1
(22) Date of filing: 14.06.2000
(51) Int. Cl.: A61K 31/352, A61K 38/00, A61K 48/00, A61P 35/00, A61K 31/35

(54) **CANCER THERAPY**
KREBSTHERAPIE
THERAPIE ANTICANCEREUSE

(30) Priority: 14.06.1999 NZ 33625999
(43) Date of publication of application: 27.03.2002
(73) Proprietor: Cancer Research Technology Limited, London WC2A 3NL (GB)
(72) Inventor: KRISSANSEN, Geoffrey Wayne, St Johns Auckland (NZ); KANWAR, Jagat, Rakesh, Mount Roskill Auckland (NZ); CHING, Lai-Ming, West Harbour Auckland (NZ)
(74) Representative: Wright, Andrew John
(86) International application number: PCT/NZ2000/000098
(87) International publication number: WO 2000/076497

(56) References cited:
- EP-A- 0 278 176
- EP-A- 0 326 149
- EP-A- 0 385 467
- EP-A- 0 488 718
- DE-A- 19 721 211
- US-A- 5 863 904
- DATABASE WPI Section Ch, Week 199716 Derwent Publications Ltd., London, GB; Class B01, AN 1997-175702 XP002233615 & JP 09 040690 A (OKA K), 10 February 1997 (1997-02-10)
- MCLACHLAN G ET AL: "The potential of cyclosporin A as an anti-tumour agent." INTERNATIONAL JOURNAL OF IMMUNOPHARMACOLOGY. ENGLAND 1990, vol. 12, no. 5, 1990, pages 469-479, XP009005663 ISSN: 0192-0561
- CAO Z. ET AL.: 'Thalidomide increases both intra-tumoural tumour necrosis factor-alpha production and anti-tumour activity in response to 5,6-dimethylxanthenone-4-acetic acid' BRITISH JOURNAL OF CANCER vol. 80, no. 5/6, 1999, pages 716 - 723, XP001080529
- FUJII H. ET AL.: 'Vaccination with B7-1+ tumor and anti-adhesion therapy with RGD pseudo-peptide (FC-336) efficiently induce anti-metastatic effect' CLINICAL & EXPERIMENTAL METASTASIS vol. 16, 1998, pages 141 - 148, XP001079183
- PEDLEY R.B. ET AL.: 'Ablation of colrectoral xenografts with combined radioimmunotherapy and tumor blood flow-modifying agents' CANCER RESEARCH vol. 56, 1996, pages 3293 - 3300, XP001080532
- ZITVOGEL L.: 'Interleukin-12 and b7.1 co-stimulation cooperate in the induction of effective antitumor immunity and therapy of established tumors' JOURNAL OF IMMUNOLOGY vol. 26, 1996, pages 1335 - 1342, XP001079493
- LISSONI P. ET AL.: 'Neuroimmunotherapy of advanced solid neoplasms with single evening subcutaneous injection of low-dose interleukin-2 and melatonin: Preliminary results' EUROPEAN JOURNAL OF CANCER vol. 29A, no. 2, 1993, pages 185 - 189, XP001080531
- HORNUNG R.L. ET AL.: 'Augmentation of natural killer activity, induction of IFN and development tumor activity during the successful treatment of established tumor murine renal cancer using flavone acetic acid and IL-2' THE JOURNAL OF IMMUNOLOGY vol. 141, no. 10, 1988, pages 3671 - 3679, XP000050669
- NAWROCKI S. AND MACKIEWICZ A.: 'Genetically modified tumour vaccines - whe we are today' CANCER TREATMENT REVIEWS vol. 25, 1999, pages 29 - 46, XP001000996
- THRASH-BINGHAM C.A. AND TARTOF K.D.: 'aHIF: A natural antisense transcript overexpressed in human renal cancer during hypoxia' THE JOURNAL OF THE NATIONAL CANCER INSTITUTE vol. 91, no. 2, 1999, pages 143 - 151, XP001080530

## Description

### FIELD OF THE INVENTION

This invention is directed to the use of therapeutic agents in combination to combat cancer. In particular it is directed to combinations of therapeutic agents which are effective against advanced and large tumour burdens.

### BACKGROUND TO THE INVENTION

Advanced cancers and large tumour burdens are refractory to treatment with therapeutic agents. Although these same agents may be effective against smaller tumours, their use does not achieve complete eradication of large tumour burdens. Large tumours can continue to grow unchecked, or their re-growth is not recognised by the body's immune system.

In addition, tumours acquire defensive and survival functions which limit the efficacy of therapeutic agents and/or the body's own immune response. For unknown reasons large tumour burdens appear to either impair or retard the generation of anti-tumour cytotoxic T lymphocyte responses. In immunotherapy, gene transfer of T cell co-stimulatory cell adhesion molecules is effective against only very small tumours and only weak anti-tumour systemic immunity is generated.

It is an object of the present invention to provide a therapeutic combination that will at least partially overcome the resistance of large tumour burdens to immunotherapy, or at least provide the public with a useful choice in the treatment of cancer.

### SUMMARY OF THE INVENTION

Accordingly, the invention provides the use of an immunotherapeutic agent in conjunction with a tumour growth restricting agent, for the manufacture of a medicament for the treatment of mammals with advanced or large tumour burdens, wherein the tumour growth restricting agent potentiates the activity of the immunotherapeutic agent and is one of 5,6-dimethylxanthenone-4-acetic acid (DXMAA) and flavone acetic acid (FAA) and wherein the immunotherapeutic agent comprises a T-cell co-stimulatory cell adhesion molecule (CAM) or a mammalian expression vector containing DNA which encodes a T-cell co-stimulatory CAM, wherein the CAM is B7.1.

As used herein, the term "immunotherapeutic agent" means a preparation which when administered to the patient results in a systemic anti-tumour immune response.

Preferably, the preparation will contain DNA, and typically, the immunotherapeutic agent will be a pharmaceutically acceptable formulation of DNA to be injected into the tumour at one or more sites so as to confer properties on the tumour tissue which generate a systemic anti-tumour immune response.

As used herein, the term "tumour growth restricting agent" means an agent which restricts or prevents tumour growth in a patient through reducing blood flow to tumours, including by inhihiting or preventing angiogenesic. Such an agent may also have other anti-tumour\immunoregulatory activities in addition to reducing blood flow.

The immunotherapeutic agent will contain DNA encoding a T cell co-stimulatory cell adhesion molecule (CAM), in a suitable expression vector. The CAM will be B7.1.

The tumour growth restricting agent is flavone acetic acid (FAA), or an analogue of xanthenone-4 acetic acid (XAA). The XAA analogue (5' 5,6-dimethylxanthenone-4-acetic acid (DMXAA).

Preferably, the immunotherapeutic agent is administered prior to administration of the tumour growth restricting agent. More preferably, the immunotherapeutic agent is administered from 12 to 48 hours prior to administration of the tumour growth restricting agent. Most preferably, administration of the immunotherapeutic agent occurs approximately 24 hours prior to administration of the tumour growth restricting agent.

In preferred embodiments, the use of the present invention may further include the administration of an additional tumour growth restricting agent. This agent may be an agent which disrupts the expression or activity of hypoxia-inducible factor-1 (HIF-1). This may conveniently be achieved by anti-sense therapy.

### DESCRIPTION OF THE DRAWINGS

While the invention is broadly defined as above, those persons skilled in the art will appreciate that it is not limited thereto and that it also includes embodiments of which the following description provides examples, In addition, the present invention will be better understood from reference to the accompanying drawings in which:

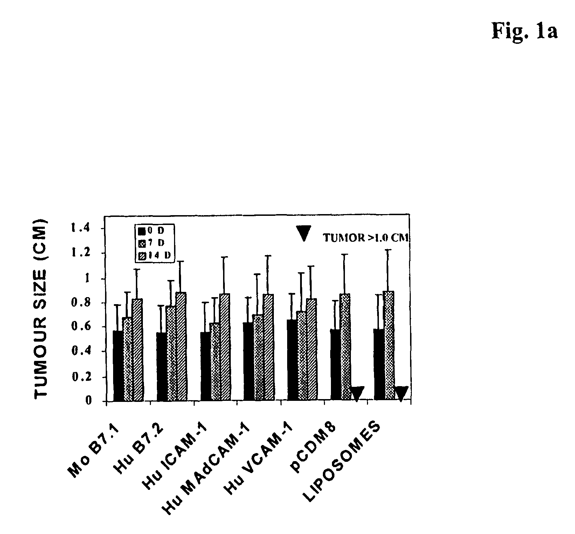

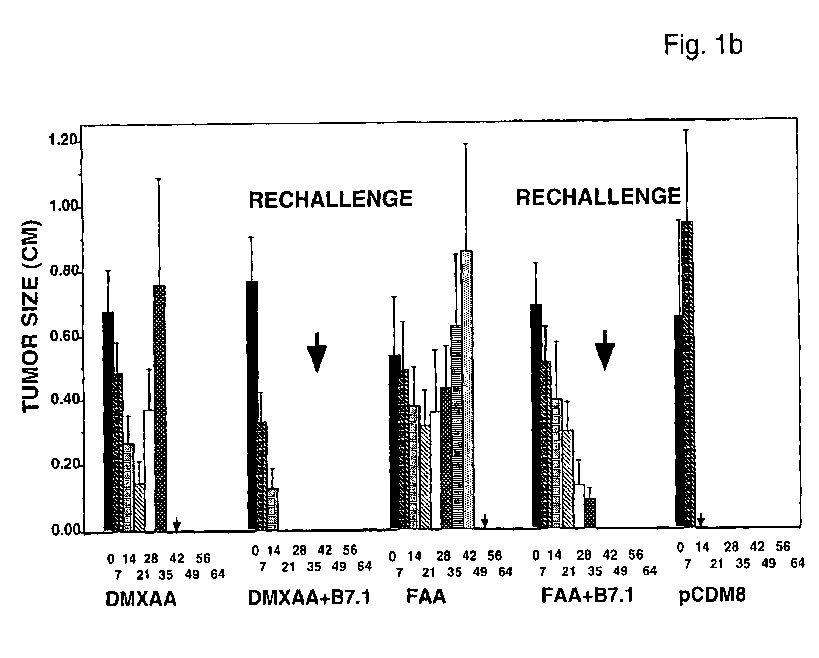

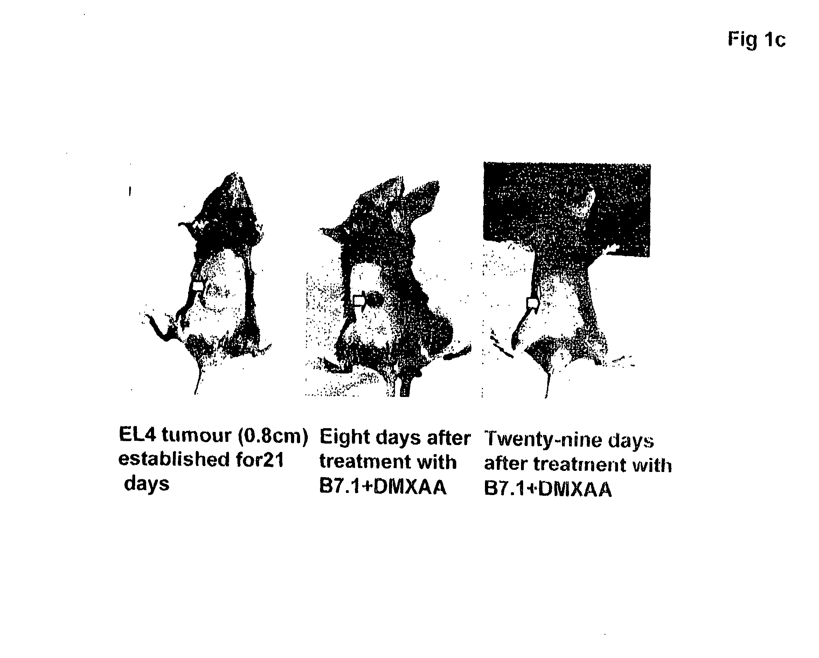

**Figure 1**. Combining the drugs DMXAA or FAA with B7.1 immunogene generates potent anti-tumour systemic immunity, whereas monotherapies are uneffective. (A) CAM gene transfer is unable to cause the rejection of large tumours. Established tumours 0.5 cm in diameter were injected with DOTAP liposomes containing 60 µg of B7.1, B7.2, ICAM-1, MAdCAM-1, and VCAM-1 cDNA. Control animals received 60 µg of empty pCDM8 vector, or liposomes. Gene transfer of each CAM slowed tumour growth, but ultimately tumours grew unchecked, and animals had to be euthanased. (B) Combining the drugs DMXAA or FAA with B7.1 immunogene eradicates large tumours. Animals bearing 0.6-0.8 cm tumours were injected i.p. with DMXAA or FAA at 300 mg/Kg and 25 mg/Kg of body weight in a volume of 0.01 ml/g body weight, respectively. For animals receiving combinational treatments, tumours were injected with DOTAP liposomes containing 60 µg of B7.1 cDNA, and DMXAA or FAA were administered 24 h later. Control animals received 60 µg of empty pCDMB vector, or liposomes alone, as indicated. The size (cm) of tumours was monitored for 42 days following gene transfer. Mice were euthanased if tumours reached more than 1 cm in diameter (denoted by small vertical arrows). The experiment was repeated twice. Mice that were cured of their tumours were rechallenged (large vertical arrow) after 42 days with 10⁶ parental tumour cells, and mice monitored for tumour regrowth for a further 22 days. (C) Photograph of mice with established and treated tumours. Illustrated is a mouse bearing a large (0.8 cm) established EL-4 tumour, and mice bearing similar sized tumours 8 and 29 days following treatment with the combination of B7.1 and DMXAA.

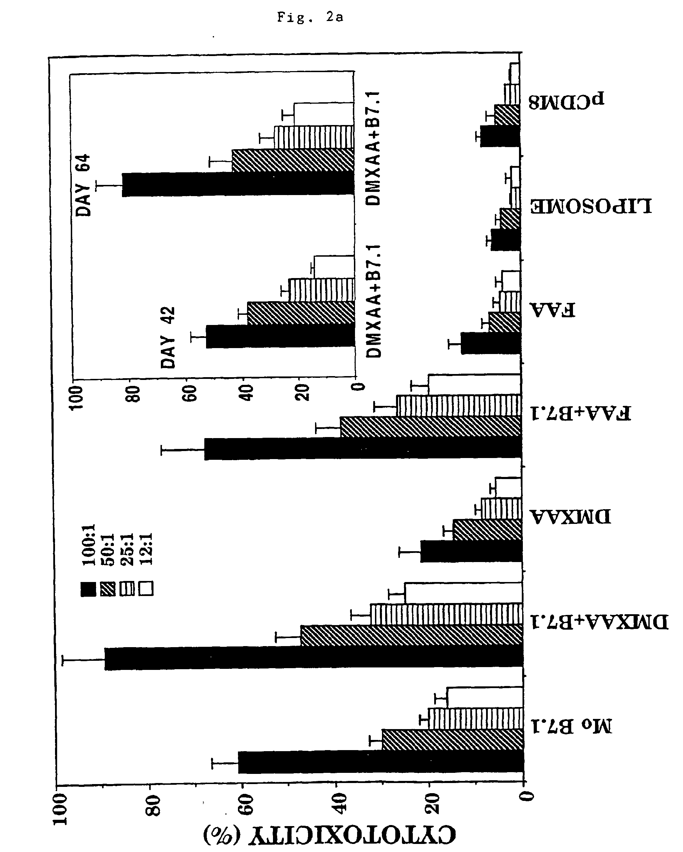

**Figure 2.** Combining B7.1 immunotherapy with DMXAA therapy generates increased CTL activity, which can be adoptively transferred to eradicate tumours. (A) Comparison of anti-tumour CTL activity generated by the different treatment regimes. Splenocytes were removed from animals 21 days following the different treatment regimes, and were tested for cytolytic activity against EL-4 tumour cells. The percent cytotoxicity is plotted against various effector to target (E:T) ratios. Control animals received empty pCDM8 vector or liposomes alone. The insert illustrates the cytolytic activity of splenocytes harvested from animals 42 days after treatment with B7.1-DMXAA, and a further 22 days later (day 64) following a rechallenge with parental EL-4 tumour cells. (B) Eradication of established tumours by adaptive transfer of anti-tumour CTL from treated mice. Splenocytes (2 x 10⁸) were adoptively transferred by intratumoral and ip. injection, from treated and control mice to recipient mice bearing established tumours (~0.6 cm in diameter). Each bar represents the mean + SD of results from 5 or 6 mice.

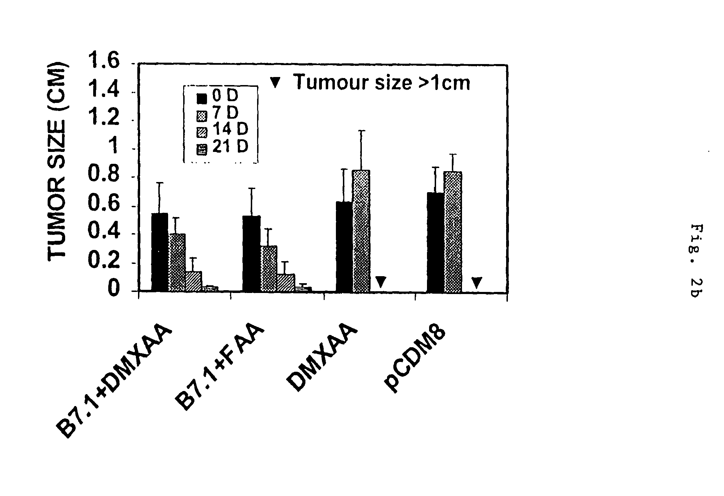

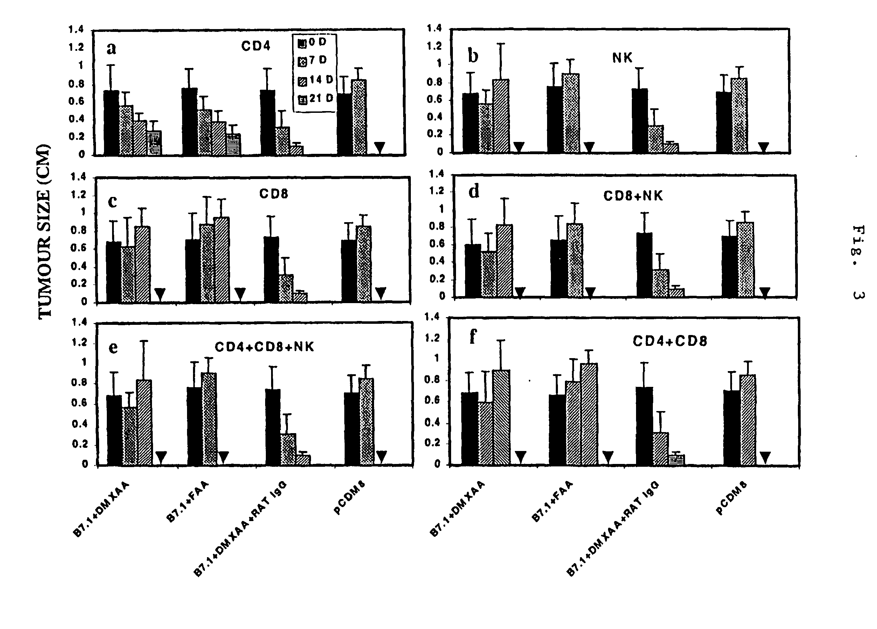

**Figure 3.** Anti-tumour immunity is largely mediated by CD8+ T cells and NK cells. Tumours (~0.6 cm diameter) were established in mice, and the contribution of leukocyte subsets to combination therapy was examined by antibody blockade. Four days prior to treatment and every alternate day for the duration of the experiment, mAbs were administered against (a) CD4 (GK1.5 mAb); (b) NK cells (PK136 mAb); (c) CD8 (53-6.72 mAb); (d) CDS and NK cells; (e) CD4, CD8, and NK cells; and (f) CD4 and CDS. Each panel (a-f) includes a control experiment in which the anti-leukocyte blocking mAb(s) was substituted with rat IgG. Mice were killed if tumours reached more than 1 cm in diameter (denoted by vertical arrows). Each bar represents the mean + SD of results from 5 or 6 mice.

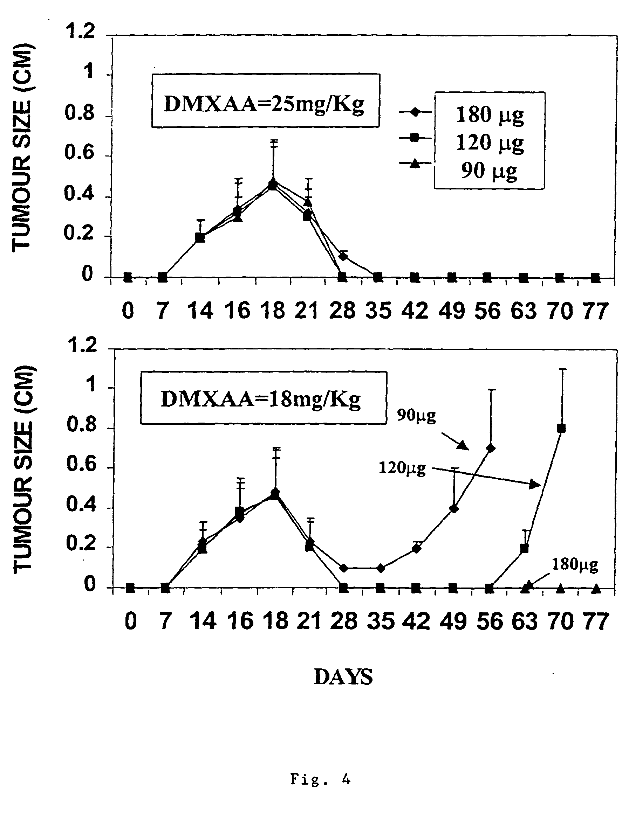

**Figure 4.** Combination therapy obviates the narrow range of therapeutic reagent dosages required for effective therapy. Tumours (~0.5 cm diameter) were established after 17 days, and injected with different amounts of B7.1 cDNA (90-180 µg). Twenty-four hours later, DMXAA was administered intraperitoneally at 25 mg/Kg body weight (upper panel), and 18 mg/Kg (lower panel). Each bar represents the mean + SD of results from 5 or 6 mice.

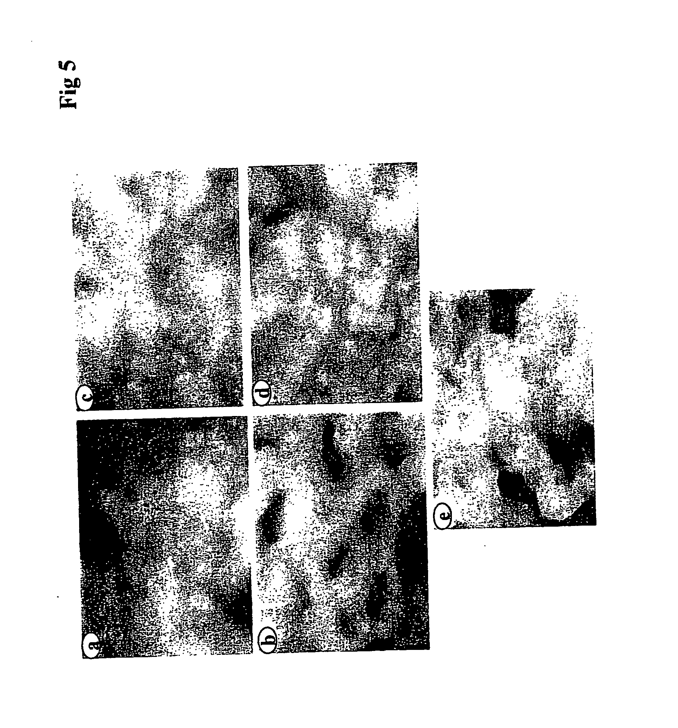

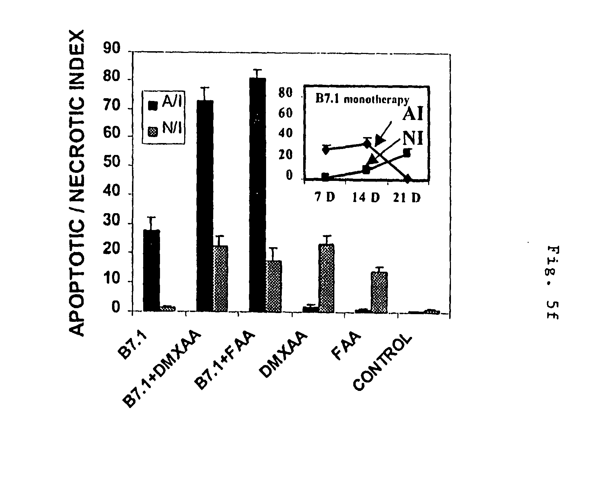

**Figure 5.** The mechanism of tumour cell death in response to B7.1 versus DMXAA therapy is different. Sections from established tumours were prepared 7 and 21 days following treatment, stained by TUNEL analysis for apoptotic cells (green fluorescent cells showing condensed fragmented nuclei), and counter-stained with propidium iodide (orange) to reveal necrotic cells, x 100. Illustrated are representative sections (a) 7 days following B7.1 treatment; (b) 21 days after B7.1 treatment; (c) 7 days after B7.1-DMXAA combination therapy; (d) 7 days after DMXAA administration; and (e) 7 days after injection of empty control vector. Tumour cell apoptosis in response to B7.1 monotherapy was followed by necrosis as revealed by the apoptotic (AI) and necrotic (NI) indices (f), whereas DMXAA monotherapy was not preceded by tumour cell apoptosis at the times examined.

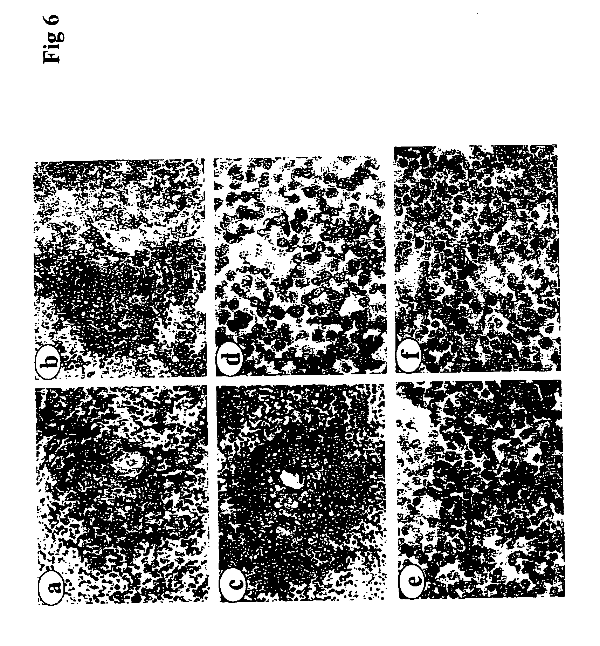

**Figure 6.** Vascular attack and B7.1 therapies induce the upregulation of tumour heat shock proteins. Immunohistochemical detection of hsp70 expression in tumours 7 days following administration of (a) DMXAA, x 40; (b) B7.1-DMXAA combination, x 40; (c) B7.1 monotherapy, x 40; (d) B7.1-DMXAA combination, x 60; (e) empty vector, x 60; and (f) sections as in (b) were also stained with a control rat IgG as primary antibody.

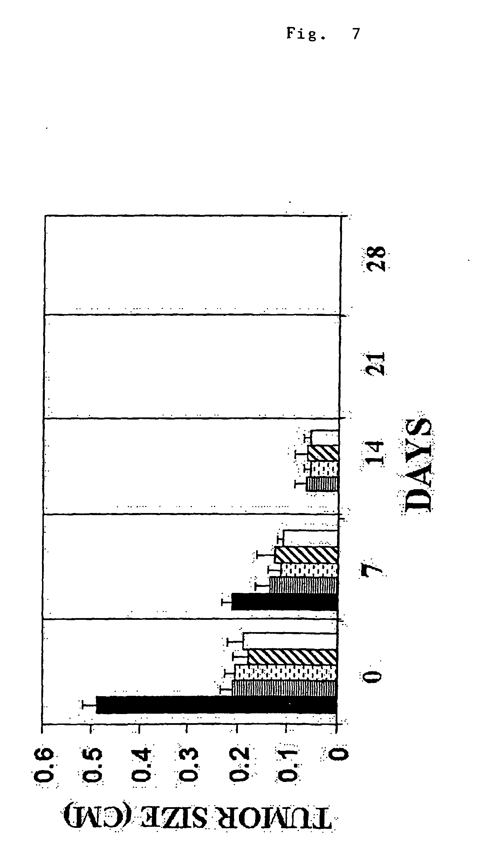

**Figure 7.** Treatment of a single tumour nodule leads to the eradication of multiple distant tumour nodules. A large single tumour (~0.5cm diameter was established in one flank, and four smaller tumours of ~0.2cm in diameter in the other flank. Gene transfer of B7-1 cDNA expression plasmid into the larger tumour, followed by systemic DMXAA therapy led to the rejection of all five tumours. Mice remained tumour-free for 35 days. For whatever reason, if the injected tumour was not larger than the non-injected tumour nodules, then tumour growth was only retarded.

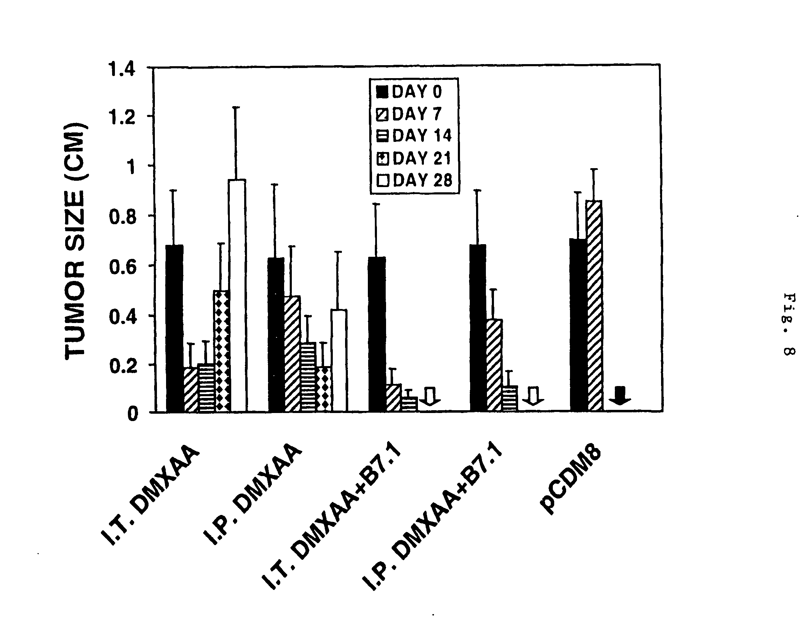

**Figure 8.** Intratumoral (IT) injection of B7-1, followed by intratumoral injection of DMXAA. The normal dosage of DMXAA (25 mg/kg) was injected. Intraperitoneal (IP) administration of DMXAA is included as a control Open arrows denote tumours eradicated, and closed arrows denote animals euthanised.

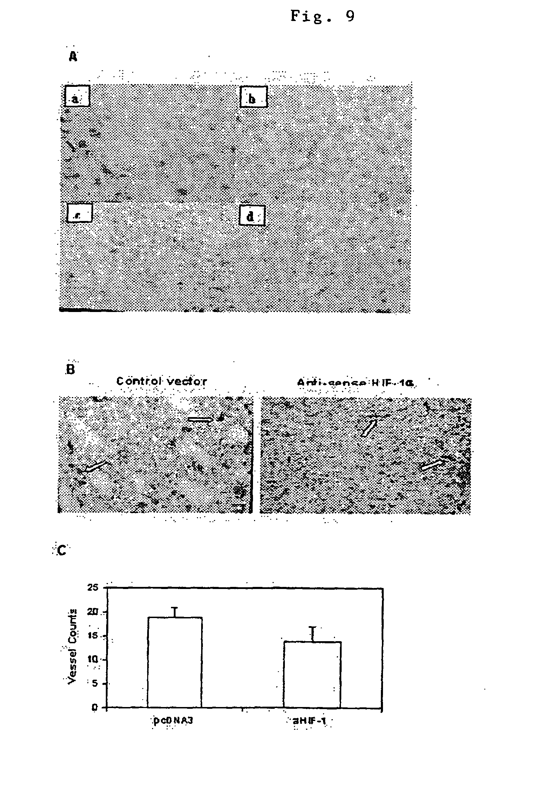

**Figure 9.** Antisense HIF-1α therapy downregulates the expression of HIF-1 and VEGF, and inhibits the formation of tumour blood vessels. (A) Down-regulation of HIF-1 and VEGF by antisense HIF-1α therapy. Tumours 0.1 cm in diameter were injected with DOTAP liposomes containing either empty vector (a, c), or antisense HIF-1α cDNA (b, d). Illustrated are representative tumour sections prepared 4 days following gene transfer, stained brown with mAbs against HIF-1α (a, b), and VEGF (c, d). (B) Antisense HIF-1α therapy blocks the formation of new tumour blood vessels. (a) Illustrated are sections prepared from 4 cm tumours injected 4 days earlier with either empty vector (pcDNA3), or antisense HIF-1α. Endothelial cells within sections were stained with the anti-CD31 mAb, revealing tumour blood vessels (examples denoted by arrows). (C) Measurement of blood vessel density. Blood vessels stained with the anti-CD31 mAb were counted in blindly chosen random fields to record mean vessel density in tumours.

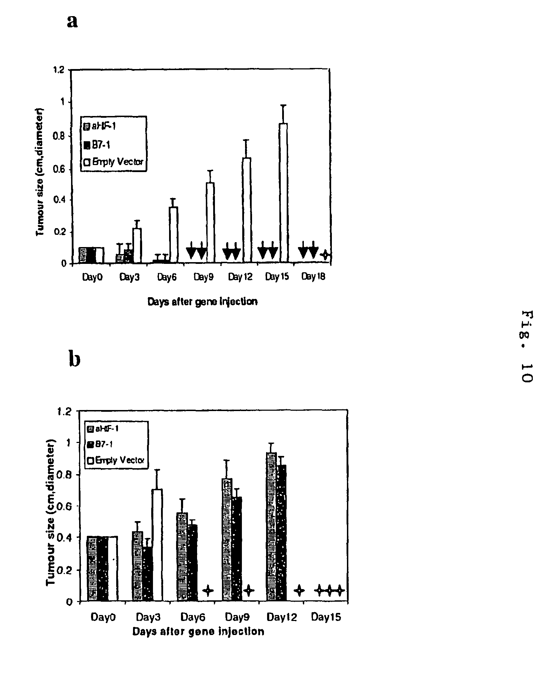

**Figure 10.** Monotherapies utilizing antisense HIF-1α anti-angiogenic therapy, and B7-1-mediated immunotherapy, are only effective against small tumours. Established tumours approximately 0.1 (a) and 0.4 (b) cm in diameter, were injected at day 0 with DOTAP liposomes containing either B7-1 cDNA, antisense HIF-1α cDNA; or empty vector in the case of control animals. The sizes (cm) of tumours was recorded following gene transfer. Complete tumour regression is denoted by vertical arrows. Mice were euthanased if tumours reached more than 1 cm in diameter (denoted by stars).

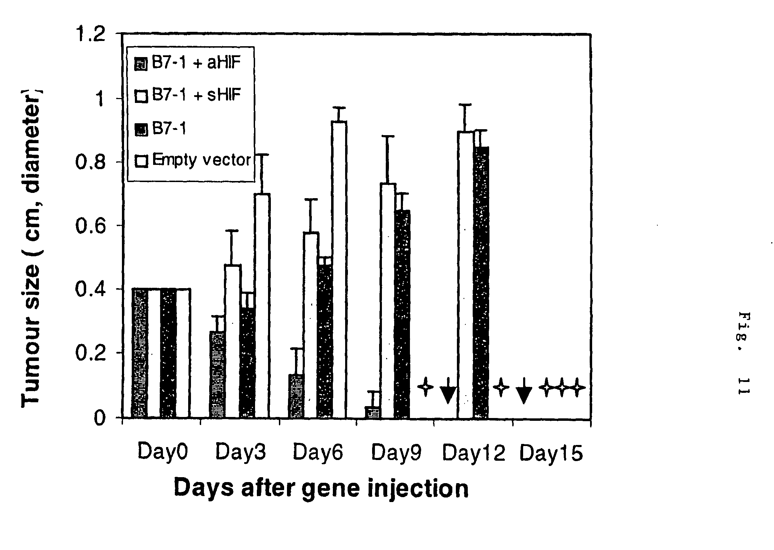

**Figure 11**. Combining antisense HIF-1α therapy with B7.1 immunotherapy causes the rapid rejection of large tumours. Tumours 0.4 cm in diameter were injected with DOTAP liposomes containing B7-1 DNA, followed 48 h later by either antisense (aHF) or sense (sHF) HIF-1α cDNA. Control animals received empty vector. The sizes (cm) of tumours was recorded following gene transfer. Mice were euthanased if tumours reached more than 1 cm in diameter (denoted by stars). Complete tumour regression is denoted by vertical arrows. Cured mice were rechallenged with 1 x 10⁶ parental tumour cells, but developed no tumours during the 2 months they were monitored (data not shown).

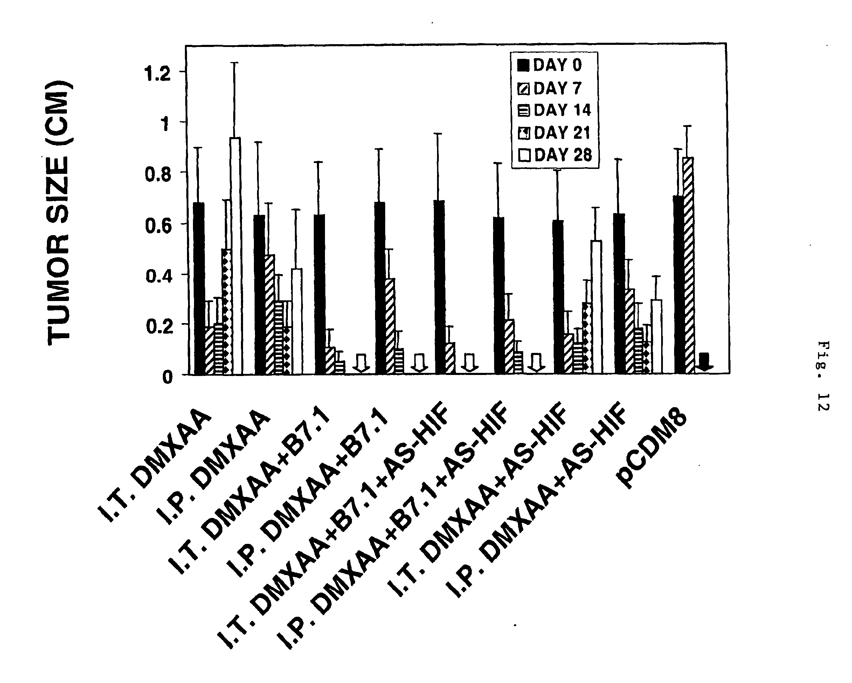

**Figure 12.** Shows results achieved using triple treatment (DMXAA + B7.1 + anti-sense HIF-1 therapy), versus other treatment regimes as shown. The triple treatment caused the most rapid eradication of tumour. Anti- reagents administered alone or together with each other were not effective. I.T. = intratumoral; LP. = intrapentoneal.

### DESCRIPTION OF THE INVENTION

As outlined above in broad terms, the present invention provides a use according to claim 1 for the manufacture of a medicament for the treatment of patients with advanced or heavy tumour burdens.

It has been noted that advanced tumour gowth is accompanied by the ability of the tumours to acquire unknown mechanisms by which they may resist the body's systemic anti-tumour immune response. Although the admmistration of chemotherapeutic agent or other means of cancer therapy may initially cause regression in the growth of the tumours, the body's immune response is unable to prevent or limit the re-growth of tumourgenic tissue that has not been eradicated from the body.

The applicants have now determined that by combining methods of immunotherapy with methods of chemotherapy previously demonstrated to be ineffective in the long term treatment of advanced or heavy tumour burdens, regression of tumours is combined with the stimulation of a strong systemic anti-tumour immune response.

The two therapeutic agents employed therefore operate in a synergistic manner to provide a combined effect which exceeds that predictable from the known properties of each.

Optimized gene transfer of T-cell co-stimulatory cell adhesion molecules (CAM), including B7.1, B7.2, and xenogeneic: (human) forms of the integrin ligands VCAM-1, MAdCAM-1, and ICAM-1 has been shown to cause rapid and complete rejection of established small tumours. Prolonged systemic anti-tumour immunity is generated, whereas other cell adhesion molecules such as human E-cadherin have only a weak ability to slow tumour growth. However, CAM-mediated immunotherapy is problematic in that it is effective against only small tumours and it generates only weak anti-tumour systemic immunity. Larger tumour burdens are able to either impair or retard the generation of anti-tumour cytotoxic T lymphocytes (CTL) rendering the tumours resistant to immunotherapy.

The anticancer agents flavone acetic acid (FAA) and 5,6-dimethylxanthenone-4-acetic acid (DMXAA) cause initial reductions in tumour size when administered, but tumours subsequently grow unchecked, and both reagents generate a weak and ineffective anti-tumour CTL response. DMXAA and FAA appear to exert their anti-tumour activities via several pathways including reduction of tumour blood flow leading to hemorrhagic necrosis and the induction of multiple immunomodulatory factors including cytokines, nitric oxide, and activated natural killer cells. However, neither agent is able to generate the desired anti-tumour systemic immunity, and they are ineffective against large tumour burdens.

The finding made by the applicants that administration of these agents in combination is effective to both eradicate advanced or large tumours and to generate anti-tumour systemic immunity is therefore surprising and representative of a significant advance in cancer treatment.

The immunotherapeutic agent can be, or include, DNA (usually cDNA) encoding AF065896). Such cDNA's can be synthesised or obtained from commercial or other sources. B7.1 can be sourced from Dr P Linsley, Bristol-Myers-Squibb, Seattle, Washington, USA.

In preferred embodiments of the invention, the immunotherapeutic agent will be administered in the form of a mammalian expression vector. While any such vector available to the skiled artisan may be selected, typical vector include expression plasmids such as pCDNA3 and pCDM8. and adenoviral- and retroviral-based vectors (such as pLXSN and pLNCX).

Alternatively, the immunotherapeutic agent may be administered directly, in a form other than in a mammalian expression vector, that is, it is not essential that the immunotherapeutic agent be administered using gene therapy. For example, T cell costimulatory CAM proteins that could be attached to the cell surface could be administered systemically.

The tumour growth restricting agent will be FAA, or a functional analogue of XAA; DMXAA.

HIF-1 is a transcription factor responsible for sensing hypoxia, and switching on hypoxia-inducible genes that stimulate the production of tumour blood vessels. Thus, in one preferred embodiment, a use comprise administration of the administration of the immunotherapeutic agent B7.1 or an expression vector encoding it, in combination with administration of an expression vector encoding an anti-sense version of HIF-1.

The immunotherapeutic agent and the tumour growth restricting agent may be administered in any suitable form, using formulations for each agent already known in the art. The administrable form and dosage required will depend on the particular immunotherapeutic agent and tumour growth restricting agent chosen for use in the present invention.

For example, when the tumour growth restricting agent is DMXAA, the DMXAA is preferably administered at the lowest effective dose. In a particularly preferred embodiment, DMXAA is injected directly into the tumour tissue. The applicants have also found in this regard that injection of DMXAA directly into the tumour can reduce the effective dose required.

The applicants have also found that a further tumour growth restricting agent (particularly an anti-angiogenic agent) can advantageously be administered in addition to the immunotherapeutic agent and first tumour growth restricting agent, to provide an additive therapeutic effect. For example, in one preferred embodiment of the invention, administration of an expression vector encoding the immunotherapeutic agent B7.1 and the tumour growth restricting agent DMXAA is combined with anti-sense therapy against HIF-1.

Also, when the tumour growth restricting agent is DMXAA and this is injected directly into the tumour, it is preferred that another less toxic anti-augiogenic reagent be administered simultaneously and systemically.

Aspects of the invention will now be described with reference to the following experimental section which is exemplary only.

### EXPERIMENTAL

### Materials and Methods

### Mice and cell lines

Female C57BL/6 mice, 6-9 weeks old, were obtained from the Animal Resource Unit, School of Medicine and Health Science, University of Auckland, Auckland. The EL-4 thymic lymphoma and mouse Lewis lung carcinoma cells (LLC) (H-2b) were purchased from the American Type Culture Collection (Rockville, MD). These cell lines were cultured *in vitro* at 37°C in DMEM medium (Gibco BRL), supplemented with 10% foetal calf serum, 50 U/ml penicillin/streptomycin, 2 mM L-glutamine, 1 mM pyruvate.

**Experimental Tumour Model**. Tumours were established by subcutaneous injection of 2 x 10⁵ and cell into the left flank of mice, and growth determining by measuring two perpendicular diameters. Animals were enthanized when tumours reached more than 1 cm in diameter, in accord with Animal Ethics Approval (University of Auckland). EL-4 and LLC tumours reached 0.6-0.9 cm in diameter after approximately 21 and 14 days, respectively. An experiments included 5 or 6 mice per treatment group, and each experiment was repeated at least once.

**Administration FAA and DMXAA Analogues**. FAA was gifted from the Department of Health & Human Services, Drug Synthesis & Chemistry laboratory, National Cancer Institute, Bethesda, USA. The sodium salt of DMXAA was synthesized in the Auckland Cancer Society Research Centre, School of Medicine and Health Science, University of Auckland. Solutions of FAA and DMXAA in 5% (w/v) sodium bicarbonate and water, respectively, were prepared fresh for each experiment and protected from light. FAA and DMXAA were injected ip. at 300 mg/Kg and 25 mg/Kg of body weight in a volume of 0.01 ml/g body weight, respectively.

**Gene Transfer of B7.1**. Complementary DNA encoding full-length human VCAM-1 was purchased from R&D Systems, Abingdon, UK; human B7-2 (Freeman *et al*, 1993) was provided by Dr G. Freeman, Dana Farber Cancer Institute, Boston, MD; human ICAM-1 was donated by Dr J. Ni, Human Genome Sciences Inc., Rockville, MD; mouse B7.1 (Chen *et al*, 1992) was provided by Dr P. Linsley, Bristol-Myers-Squibb, Seattle, WA. We have previously reported the cloning and characterization of human MAdCAM-1 cDNA (Leung *et al,* 1996). The CAM pCDM8 expression vectors were prepared by cesium chloride gradient centrifugation, and diluted to 600 µg/ml in a solution of 5% glucose in 0.01% Triton X-100. They were misced in a ratio of 1:3 (wt:wt) with DOTAP cationic liposomes (Boehringer Mannheim., Germany). Tumours were injected with 100 µl of DNA (60 µg/liposome complexes, unless otherwise staled.

**Combining B7.1-mediated Immunotherapy with FAA/DMXAA-Mediated Vasculature Attack**. Tumours, 0.6 to 0.9 cm in diameter, were injected at multiple sites with 100 µl of DNA (60 µg)/liposome complexes. Twenty-four hours later, DMXAA or FAA were administered i.p. as described above. Treated mice that remained tumour-free were rechallenged 6 weeks after administration of FAA or DMXAA and B7.1, by s.c. injection of EL-4 cells and LLC cells (0.1 ml) in the opposing flank (right flank).

**Measurement of the Generation of Anti-Tumour CTL**. Splenocytes were harvested 21, and 42 days following initial gene transfer, and 22 days following a parental tumour challenge. They were incubated at 37°C with EL-4 target cells in graded E:T ratios in 96-well round-bottom plates. After a 4-hour incubation, 50 µl of supernatant was collected, and lysis was measured using the Cyto Tox 96 Assay Kit (Promega, Madison, WI, USA). Background controls for non-specific target and effector cell lysis wens included. After background subtraction, percentage of cell lysis was calculated using the formula: 100 x (experimental-spontaneous effector-target spontaneous target/maximum target-spontaneous target).

**Adoptive Transfer of Anti-Tumour** CTL. Adoptive transfer of anti-tumour splenocytes was as described previously (Kanwar *et al*, 1999). Briefly, splenocytes obtained from mice 21 days following therapy were resuspended in Hank's balanced salt solution containing 1% FCS, and stimulated with 5 µg/ml PHA and 100 U/ml recombinant mouse IL-2 for 4 to 5 days. Animals bearing tumours 0.6 cm in diameter received both intratumoral and i.p. injections of 2 x 10⁸ cultured splenocytes.

**Depletion of Leukocyte Subsets**. Mice were depleted of CD8+, and CD4+ T cells and NX cells by ip. and i.v. injection 4 days prior to gene transfer, and thereafter every alternate day with 300 µg (0.1 ml) of the 53-6.72 (anti-CD8), Gk1.5 (anti-CD4), and PK136 (anti-NK) mAbs. Rat IgG (Sigma, USA) was used as a control antibody. Antibodies were an ammonium sulphate fraction of ascites, which titered to at least 1:2,000 by FACS (Becton Dickinson & Co., CA, USA) staining splenocytes. Depletion of individual leukocyte subsets was found to be more than 90% effective, as determined by FACScan analysis. Rat hybridomas secreting mAbs against mouse CD8 (53-6.72 mAb), CD4 (Gk1.5 mAb), and NK cells (PK136 mAb) were purchased from the American Type Culture Collection, Rockville, MD, USA.

**In Situ Detection of Apoptotic Cells**. Tumours were excised and immediately frozen in dry ice, stored at -70°C, and serial sections of 6 µm thickness prepared. TUNEL staining was performed using an *In Situ* apoptosis detection kit from Boehringer Mannheim, Germany. Briefly, frozen sections were fixed with paraformaldehyde solution (4% in PBS, pH 7.4), and permeabilized with a solution containing 01% Triton X-100 and 0.1% sodium citrate. After washing they were incubated with 20µl TUNEL reagent for 60 min at 37°C, and examined by fluorescence microscopy. Some slides were counterstained with propidium iodide (P1; Sigma, CA, USA) to distinguish necrotic cells from those undergoing apoptosis. Adjacent sections were conuterstained with haematoxylin and cosin and mounted. The total number of apoptotic or necrotic cells were counted. The apototic index (AI) or necrotic index (NI) was calculated as follows: Al or N1 = number of apoptotic or necrotic cells x 100/total number of nucleated cells.

**Immunohistology**. Tumours were excised on the seventh day following administration of therapeutic agents and controls, immediately frozen in dry ice, stored at -70°C in isopentane, and sections of 10 µm thickness prepared. Sections were mounted on poly L-lysine-coated slides, and endogenous peroxidases blocked by incubating slides for 30 min 0.3% H₂O₂ in methanol. Sections were stained with mouse anti-Hsp70 mAb (SPA-810 antibody; StressGen Biotechnologies Corp., Victoria, Canada) using a mouse-on-mouse immmunodetection ABC Elite peroxidase kit (Vector Laboratories, Burlingame, CA, USA). They were developed with SIGMA FAST DAB (3, 3'-diaminobenzidine tetrahydrochloride) with metal enhancer CoCl₂ tablets, and counterstained with haematoxylin and eosin. As a control, the primary antibody was substituted with rat IgG (Sigma, USA).

**Statistical analysis.** All experiments performed in vivo were repeated at least once with similar results obtained. Results were expressed as mean values + standard deviation (S.D.), and a Student's t test was used for evaluating statistical significance. A value of p<0.05 denotes statistical significance, whereas p<0.001 denotes results that are highly significant.

### RESULTS

**CAM Gene Monotherapy is Unable to Check the Growth of Large Tumours.** EL-4 cells (2 x 10⁵) subcutaneously implanted into mice grow rapidly, forming a solid tumour 1 cm in diameter within 4 weeks. We have previously demonstrated that small EL-4 tumours (0.1-0.3 cm diameter) transfected in situ with B7.1, B7.2, VCAM-1, and ICAM-1 cDNA failed to grow, and mice remained tumour-tree for at least two months (Kanwar et al, 1999). In contrast, as shown in Figure 1*a*, larger tumours (>0.5cm) are refractory to treatment in response to B7.1 and several other costimulatory CAMs. Tumour growth is slowed, but ultimately the tumour grows unchecked.

**DMXAA and FAA are Unable to Check the Growth of Large Tumours**. Systemic administration of optimal doses of DMXAA and FAA to mice bearing large EL-4 tumours (0.6-0.8 cm in diameter) led to immediate reductions in the sizes of tumours (Fig. 1*b*), accompanied by marked tumour necrosis (refer below). DMXAA was the more potent of the two reagents, causing tumours to shrink to 0.1-0.2 cm over a period of 3 weeks, whereas the tumours of FAA-treated animals were reduced to 0.2-0.4 cm in diameter. However, tumours began to grow unchecked by day 28 and animals had to be sacrificed during the sixth week.

**Combined Therapy by Timed Delivery of B7.1 and DMXAA/FAA Eradicates Large Tumours**. We hypothesized that simultaneous administration of the B7.1 pCDM8 expression vector and DMXAA/FAA might impair CAM-mediated anti-tumour immunity, as dying and necrotic tumour cells would not be able to adequately express B7.1. This notion proved correct, and hence established tumours (0.6-0.8 cm in diameter) were first treated with B7.1 to stimulate anti-tumour immunity, and DMXAA and FAA were administered one day later to retard tumour growth. Remarkably, tumours rapidly diminished in response to the combination of B7.1 and DMXAA accompanied by massive necrosis, such that by the third week of treatment tumours had completely disappeared (Fig. 1*b*). The gross tumours of DMXAA-B7.1-treated animals took on the appearance of a wound that rapidly healed forming a scab that eventually flaked off leaving perfectly healed skin. Unlike B7.1 treatment, which leaves what appears to be a palpable fibrotic core, the tumour sites of DMXAA-B7.1-treated animals were completely healed (Fig. 1*c*). Similar results were obtained with the combination of FAA and B7.1, although tumours did not completely disappear until the sixth week.

**Combined Therapy Generates Potent and Prolonged Tumour-Specific Cytolytic T cell Activity.** The anti-tumour CTL activity of splenocytes obtained from treated mice, 21 days following gene transfer, was significantly (p<0.001) augmented in animals treated with the combination of B7.1 and DMXAA, and Slightly enhanced with the combination of B7.1 and FAA, versus those receiving B7.1 monotherapy (Fig. 2a). In contrast DMXAA and FAA alone were very poor effectors, though they did enhance CTL production above that seen with empty vector and liposome controls.

Animals previously cured by combination therapy completely rejected the substantial challenge of 1 x 10⁷ parental tumour cells for the 40 days they were monitored (Fig. 1*b*, Table 1). Both DMXAA-B7.1 and FAA-B7.1 therapy provided complete protection, indicating that potent systemic anti-tumour immunity had been achieved. Anti-tumour CTL activity was still very strong 42 days following initial treatment with DMXAA-B7.1, and was stimulated further following the rechallenge with parental tumour cells (Fig. 2*a*).

We have previously reported that adoptive transfer of splenocytes, from mice whose tumours had been treated with B7.1, leads to eradication of tumours in recipients (Kanwar *et al*, 1999). In accord, adoptive transfer of 2 x 10⁸ splenocytes from B7.1-DMXAA and B7.1-FAA treated mice into recipients bearing established EL-4 tumours (up to 0.8 cm in diameter) resulted in rapid and complete tumour regression (Fig. 2*b*). Tumours larger than 0.8cm in diameter were refractory to such treatment. In contrast, splenocytes from DMXAA or empty vector treated mice displayed no detectable anti-tumour activity.

**Composition of the Leukocyte Effector Population**. *In vivo* antibody blocking studies revealed that the primary rejection of tumours in reponse to either combined DMXAA-B7.1 or FAA-B7.1 therapy was very dependent on the presence of CD8+ T cells and NK cells, but only partially dependent on CD4+ T cells. Simultaneous depletion of either CD8+ T cells and NK cells; CD4+ and CD8+ T cells; or CD4+/CD8+ T cells and NK cells, severely impaired anti-tumour immunity leading to rapid tumour growth (Fig. 3).

**Anti-Tumour Immunity is Tumour-Specific**. Similar results to the above have been obtained with the weakly immunogenic LLC. Thus, combinational B7.1-DMXAA therapy completely cured mice of subcutaneous LLC, and generated anti-tumour systemic immunity that protected all mice against challenge with 1 x 10⁵ parental tumour cells, and 80% of mice against a challenge with 1 x 10⁷ tumour cells (Table 1). However, mice cured of EL-4 tumours were unable to resist a challenge of 1 x 10⁴ LLC cells, and vice versa mice cured of LLC were not protected against a challenge with 1 x 10⁴ EL-4 cells; demonstrating that anti-tumour immununity is tumour-specific.

**Gene Dosage Effect**. Both FAA and DMXAA have an unusual 'threshold' behavior in which only a very narrow range of high doses (22.5-25 mg/Kg body weight) are active, and acceptable in terms of toxicity (Baguley *et al*, 1993; Pedley *et al*. 1996). Further, we have previously reported that B7.1 and other costimulatory CAMs display a restrictive gene dosage effect, such that gene transfer of 60 µg of B7.1/pCDM8 expression plasmid is optimal, whereas lesser or greater amounts are much less effective (Kanwar *et al*, 1999). To investigate whether a high gene dosage would impair combination therapy, tumours were injected with amounts (90-180 µg) of B7.1/pCDM8 plasmid followed by administration of an optimal dose of DMXAA (25 mg/Kg) (Fig. 4*a*). All mice rapidly rejected their tumours, and a rechallenge of 2 x 10⁵ parental EL-4 cells. Thus an identical outcome is achieved with a broad high dose range of the therapeutic gene and an optimal dose of DMXAA. In contrast, when the DMXAA concentration was suboptimal, a gene dosage effect is clearly evident, such that only large amounts (180 µg) of B7.1/pCDM8 expression plasmid could generate effective anti-tumour immunity (Fig 4*b*).

**Mechanisms for B7.1 and DMXAA-Mediated Tumour Regression.** We have previously reported that CAM-mediated anti-tumour immunity is accompanied by augmented CTL activity involving both the perform and Fas-ligand pathways, suggesting that EL-4 cells are targeted to undergo immune-mediated programmed cell death (Kanwar *et al,* 1999). This notion was confirmed by TUNEL staining of tumour sections prepared 7, 14, and 21 days after B7.1 gene transfer. Sections were counterstained with PI, which stains only the DNA of necrotic cells, allowing necrotic versus apoptotic cells to be distinguished. B7.1 immunotherapy was accompanied by marked tumour cell apoptosis (green fluorescence) at day 7, which peaked 14 days following gene transfer, and was replaced at day 21 by marked necrosis (orange fluoresence) (Fig. 5a, b and f). In contrast; there was a predominance of necrotic cells in tumour sections from DMXAA-treated mice, where very few apoptotic cells were present (Fig. 5d). Surprisingly, combination therapy increased the number of apoptotic cells compared to B7.1 monotherapy (Fig. 5c), while retaining the same degree of necrosis observed with DMXAA monotherapy. Untreated tumours showed no sign of necrosis and apoptotic cells were absent (Fig. 5e).

We hypothesized that the small increase in the generation of CTL in response to DMXAA may be an indirect effect, caused by the generation of CTL in response to heat shock proteins upregulated on stressed and dying tumour cells. Indeed, heat shock protein 70 was consistently found upregulated on tumour cells either surrounding or within the vicinity of blood vessels (Fig. 6). Similarly, hsp70 was upregulated in tumours treated with B7.1, although not necessarily in the vicinity of blood vessels. In contrast, there was no sign of heightened bsp70 expression in tumours treated with empty vector alone.

| Table: 1 Combined therapy induces tumour-specific immunity" | | | | | |
|---|---|---|---|---|---|
| Tumour cells Injected | Mice challenged with tumour cells | Challenge dose | Tumour incidence | Tumour onset (days) tumour size <0.2 cm | Time of sacrifice (days)^{b} (tumour size <1.0 cm) |
| | EL4 | 1X10⁴ | 0/6 | - | - |
| | | 1X10⁵ | 0/6 | - | - |
| | | 1X10⁷ | 0/6 | - | - |
| EL4 | | | | | |
| | | 1X10⁴ | 5/5 | 16-24 | 35±4.2 |
| | LLC | 1X10⁵ | 5/5 | 14-20 | 30±5.1 |
| | | 1X10⁷ | 5/5 | 14-19 | 29±4.3 |
| LLC | LLC | 1X10⁴ | 0/6 | - | - |
| | | 1X10⁵ | 0/6 | - | - |
| | | 1X10⁷ | 1/6 | - | 29 |
| | EL-4 | 1X10⁴ | 5/5 | 18-26 | 38±6.1 |
| | | 1X10⁵ | 5/5 | 16-22 | 36±6.1 |
| | | 1X10⁷ | 5/5 | 16-24 | 33±4.5 |

| | | | | | |
|---|---|---|---|---|---|
| ^{a}Tumors were established with EL4 or LLC tumor cells and B7.1 DNA injected intratumoraly. Twenty-four hours later, DMXAA was administered i.p. and after 21 days animals were challenged. | | | | | |
| ^{b}Values represent means ±SD, calculated from 5 or 6 mice per group. | | | | | |

### Gene transfer of an expression plasmid encoding antisense HIF-1α induces the rejection of established tumours

EL-4 tumours of 0.1 cm in diameter were established in C57BL/6 mice, and injected with a DNA/liposome transfection vehicle containing 100µg of antisense HIF-1α pcDNA3B plasmid DNA- Immunohistochemical analysis of tumour sections prepared 2 days following gene transfer, revealed antisense therapy resulted in almost the complete inhibition of HIF-1 expressed endogenously in growing tumours (Figure 9A). Tumour growth was monitored for 4 weeks following gene transfer (Figure 10a). The pattern of growth was compared to mice treated with 100µg of empty vector control Tumours grew rapidly in the control group reaching 1 cm in size 14-17 days following gene transfer, whereas tumours treated with the antisense HIF-1 α plasmid completely and rapidly regressed within one week of gene transfer. Mice remained tumour-free for a further 21 day period during which they were monitored.

### Antisense HIF-1α therapy does not eradicate large tumours, but slows their growth

We have previously demonstrated that tumours become refractory to immunotherapy once they reach 0.3 cm in diameter.¹ To determine whether antisense HIF-1α therapy was similarly ineffective against large tumours, EI-4 tumours of 0.4 cm in diameter were established, and treated with 100µg antisense HIF-1α expression plasmid. As shown in Figure 10b, none of the mice rejected their tumours, albeit there was a significant (P< 0.01) inhibition of tumour growth. All tumours eventually reached 1 cm in diameter within 2 weeks, and mice had to be euthanased.

### Vascular attack by antisense HIF-1α synergizes with B7-1 immunotherapy to eradicate large tumours

Here we consider the possibility that an anti-HIF-1α reagent might be a suitable anti-angiogenic substitute for DMXAA in combination therapy. As reported previously¹, gene transfer of a B7-1 expression plasmid into small tumours (0.1cm in diameter) (Figure 10a) led to complete tumour eradication within one week of gene transfer. There was no significant difference between the growth pattern of tumours treated with B7-1 and antisense HIF-1α (P>0.05). In contrast, large tumours (0.4cm diameter) were intractable to treatment (Figure 10b), as had been the case with antisense HIF-1α therapy. The failure of B7-1 therapy is not the result of poor transfection efficiency as B7-1 gene transfer led to expression of B7-1 in 90% of tumour cells, as reported in previous work. ¹ For combination therapy, 0.4 cm diameter tumours were first injected with DNA/liposome complexes containing 100µg B7-1, followed 48 h later by 100 µg antisense HIF-1α plasmids. Combined gene therapy led to complete tumour regression within 10 days, and mice remained tumour-free for 3 weeks (Figure 11). To determine whether systemic anti-tumour immunity had been generated, cured mice were rechallenged with 1 × 10⁶ parental tumour cells. Such mice resisted the challenge, and remained tumour-free for at least 2 months.

As a control, B7-1 immunotherapy was combined with an expression plasmid encoding the HIH-1α cDNA fragment inserted into pcDNA3 in a sense orientation In marked contrast to the above results, sense HIF-1α could not enhance the therapeutic efficacy of B7-1 (Figure 11), producing results that were not statistically (P> 0.05) different from empty vector treated control mice.

### Antisense HIF-1α therapy inhibits VEGF expression, and reduces the density of tumour blood vessels

Given that HIF-1α antisense down-regulated the expression of HIF-1α in EL-4 tumour cells, we sought to determine whether the expression of downstream effectors such as VEGF was abolished. Expression of VEGF was specically reduced in response to antisense HIF-1α (Figure 9A, compare c and d), resulting in a statistically significant (P<0.01) 30% reduction in tumour vessel density (Figures 9B and C), compared to mock treatment with empty vector. In addition tumour blood vessels present after antisense HIF-1α therapy were small and poorly formed.

### MATERIAL AND METHODS

### Mice and cell lines

Male C57BL/6 mice, 6-8 weeks old, were obtained from the Animal Resource Unit, Faculty of Medicine and Health Science, University of Auckland, Auckland, New Zealand. The EL-4 thymic lymphoma, which is of C57BL/6(H-2b) origin, was purchased from the American Type Culture Collection (Rockville, MD, USA). It was cultured at 37°C in DMEM medium (Gibco BRL, Grand Island, NY, USA), supplemented with 10% foetal calf serum, 50U/ml penicillin/streptomycin, 2 mM L-glutamine, 1mM pyruvate.

### Expression plasmids

A 320bp cDNA fragment encoding the 5'end of HIF-1 α (nucleotides 152 to 454; GenBank AF003698) was produced through PCR using the IMAGE clone 851237 as a template, and the two primers (5' GGG GAT CCT CTG GAC TTG TCT CTT TC3' and 5' GGG CTC GAG TAA CTG ATG GTG AGC CTC 3'). The fragment was cloned into pGEMT (Promega Corporation), and then subcloned into pcDNA3 (Invitrogen Company) at BamHI and XhoI sites in sense orientation, and into pcDNA3B at XhoI and BamHI sites in an antisense orientation. The pcDNA3B expression vector is identical to pcDNA3, except the polylinker is reversed (Lehnert et al., unpublished). The expression plasmid B7-1-pCDMB, which contains a 1.2 kb cDNA fragment encoding full-length mouse B7-1 was constructed from a cDNA clone provided by Dr P Linsley, Bristol-Myers-Squibb, Seattle, WA, USA.

### Gene transfer of expression plasmids and measurement of anti-tumour activity

Purified plasmids were diluted in a solution of 5% glucose in 0.01% Triton X-100, and mixed in a ratio of 1:3 (wt:wt) with DOTAP cationic liposomes (Bachringer Mannheim, Mannheim, Germany), which is an efficient transaction vehicle.¹ Final plasmid concentration was 1 mg/ml. Tumours were established by injection of 2 × 10⁵ EL-4 tumour cells into the right flank of mice, and growth determined by measuring two perpendicular diameters. Animals were killed when tumours reached more than 1 cm in diameter, in accord with Animal Ethics Approval (University of Auckland). Tumours reached 0.1cm and 0.4 cm in diameter after approximately 14-18 days, and were injected with 100 ul expression plasmid (100µg). For combinational treatment, reagents were delivered in a timed fashion, as described above for B7-1 /DMXAA combination therapy. Thus, B7-1 cDNA was injected first, followed by HIF antisense cDNA 48 h later. Empty vectors served as control reagents. Cured mice were rechallenged 3 weeks after the disappearance of tumours by injecting 1 × 10⁶ EL-4 cells subcutaneously into the opposing flank (left flank). All experiments included 6 mice per group, and each experiment was repeated at least once. Results were expressed as mean values ± standard deviation (s.d.), and a Student's *t* test was used for evaluating statistical significance. A value less than 0.05 (P < 0.05) denotes statistical significance.

### Immunohistochemistry

Tumour cryosections (10 µm) were prepared 2 days following gene transfer, treated with acetone, rinsed with PBS, and blocked with 2% BSA for 2 h. The sections underwent a overnight incubation with either a hamster anti-B7-1 mAb (1G10, Pharmingen, San Diego, CA, USA), mouse anti-mouse HIF-1α mAb (H1α67, Novus Biologicals, Inc., Litleton, CO, USA), or rabbit polyclonal antibodies against VEGF (Ab-1, Lab Vision Corporation; CA, USA). They were subsequently incubated for 30 min with appropriate secondary antibodies, using the VECTASTAIN Universal Quick kit (Vector Laboratories, Burlingame, CA, USA); and developed with Sigma FAST DAB (3,3'-diaminobenzidine tetrahydrochloride) and CoCl₂ enhancer tablets (Sigma). Sections were counterstained with Mayer's hematoxylin, mounted, and examined by microscopy.

### Assessment of vascularity

The method for assessment of vascularity was as described.³³ Briefly, 10-µm sections were cut from fresh frozen tumours 4 days following gene transfer. Slides were immunostained with the anti-CD31 antibody, MEC13.3 (Pharmingen, CA, USA), as above. Blood vessels stained with the anti-CD31 mAb were counted in blindly chosen random fields.

### INDUSTRIAL APPLICABILITY

Thus, in accordance with the present invention, the application have provided a medicament for cancer therapy which represents a significant advance over previous approaches in terms of eradication of advanced or large tumours. The advance represented by the present invention is particularly remarkable where the immunotherapeutic agent is administered in appropriate period of time prior to administration of the tumour growth restricting agent. This results m complete eradication of large tumour burdens and the generation of a potent anti-tumour systemic immunity.

### REFERENCES

1) Kanwar, J.R., Berg, R.W., Lehnert, K, and Krissansen G.W. Taking lessons from dendritic cells: Multiple xenogeneic ligands for leukocyte integrins have the potential to stimulate anti-tumour immunity. Gene Therapy, *6*: 1835-1844, 1999.
2) Hersey, P. Impediments to successful immunotherapy. Pharmacol. Ther., *81:* 111-119, 1999.
3) Griffioen, A.W., Damen, C.A, Mayo, K.H., Barendsz-Janson, A.F., Martinotti, S., Blijham, G.H., and Groeneegen, G. Angiogenesis inhibitors overcome tumour induced endothelial cell anergy. Int. J. Cancer, *80:* 315-319, 1999.
4) O' Reilly, M.S., Boehm, T-, Shing, Y., Fukai, N., Vasios, G., Lane, W.S., Flynn, E., Birkhead, J.R., Olsen, B.R., and Folkman, J. Endostatin: An endogenous inhibitor of angiogenesis and tumour growth. Cell, *88*: 277-285, 1997.
5) O'Reilly, M.S., Boehm, T., Shing, Y., Fukai, N., vasios, G., Lane, W.S., Flynn, E., Birkhead, J.R., Olsen, B.R., and Folkman, J. Angiostatin: A novel angiogenesis inhibitor that mediates the suppression of metastases by a Lewis lung carcinoma. Cell, *79*: 315-328, 1994.
6) Corbett, T.H., Bissery, M.C., Wozniak, A., Polin, J., Tapazoglou, E., Dieckman, J., and Valeriote, F. Acitivity of flavone acetic acid (NSC-347512) against solid tumours of mice. Investigational New Drugs. *4*: 207-220, 1986.
7) Shoemaker, R., Wolpert-DeFilippes, M., Plowman, J., Abbott, B., Venditti, J., Trader, M., Griswold, D., Gerlach, J., and Ling, V. Pleiotropic resistance and drug development. Progress Clin. Biol. Res., *223*: 143-149, 1986.
8) Zaharko, D.S., Grieshaber, C.K., Plowman, J., and Cradock, J.C. Therapeutic and pharmacokmetic relationships of flavone acetic acid: an agent with activity against solid tumours. Cancer Treatment Reports, *70*: 1415-1421, 1986.
9) Plowman, J., Narayanan, V.L., Dykes, D., Szarvasi, E., Briet, P., Yoder, O.C., and Paull, K.D. Flavone acetic acid: a novel agent with preclinical antitumour activity against colon adenocarcinoma 38 in mice. Cancer Treatment Reports, *70*: 631-635, 1986.
10) Bibby, M.C., and Double, J.A. Flavone acetic acid-from laboratory to clinic and back. Anti-Cancer Drugs, *4*: 3-17, 1993.
11) Baguley, B.C., and Ching, L.M. Immunomodulatory actions of xanthenone anticancer agents. BioDrugs, *8*: 119-127, 1997.
12) Freeman, G.J., Gribben, J.G., Boussiotis, V.A., Ng, J.W., Restivo, Jr. V.A., Lombard, L.A., Gray, G.S., and Nadler, L.M. Cloning of B7.2: a GTLA-4 counter-receptor that costimulates human T cell proliferation. Science, *262*: 909-911, 1993.
13) Chen, L., Ashe, S., Brady, WA, Hellstrom, I., Henstrom, K.E., Ledbetter, J.A., McGowan, P., and Linsley, P.S. Costimulation of anti-tumour immunity by the B7 counterrcceptor for the T lymphocyte molecules CD28 and CTLA-4. Cell, *71*: 1093-1102, 1992.
14) Leung, E., Greene, J., Ni, J., Raymond, L.G., Lehnert, K., Langley, R., and Krissansen, G.W. Cloning of the mucosal addressin MAdCAM-1 from human brain:identification of novel a spliced transcripts. Immunol Cell BioL, *74*:490-496,1996.
15) Baguley, B.C., Cole, G., Thomsen, L.L.. and Zhuang, L. Serotonin involvement in the antitumour and host effects of flavone-8-acetic acid and 5,6-dimethylxanthenone-4-acetic acid. Cancer Chemother. Pharmacol., *33*: 77-81, 1993.
16) Pedley, R.B., Boden. J.A., Boden, R., Boxer, G.M., Flynn, A-A., Keep, P.A., and Begent, R.H. Ablation of colorectal xenografts with combined radioimmunotherapy and tumour blood flow-modifying agents. Cancer Res., *56*:3293-3300, 1996.
17) Finlay, G.J., Ching, L.M., Wilson, W.R., and Baguley, B.C. Resistance of cultured Lewis lung carcinoma cell lines to tiazofurin. J. Nat. Cancer Inst., *79*: 291-296, 1987.
18) Ching, L.M., and Baguley, B.C. Induction of natural killer cell activity by the antitumour compound flavone acetic acid (NSC 347 512). Eur. J. Cancer Clin. OncoL, *23*: 1047-5100, 1987.
19) Clung, L.M., and Baguley, B.C. Effect of flavone acetic acid (NSC 347,512) an spleuic cytotoxic effector cells and their role in tumour necrosis. Eur. J. Cancer Clin. Oncol, *25*: 821-828, 1989.
20) Baguley, B.C., Zhuang, L., and Kestell, P. Increased plasma serotonin following treatment with flavone-8-acetic acid, 5,6-dimethylxanthenone-4-acetic acid, vinblastine, and colchicine: relation to vascular effects. Oncol. Res., *9*: 55-60, 1997.
21) Thomsen, L.L., Ching, L.M., and Baguley, B.C. 1990. Evidence for the production of nitric oxide by activated macrophages treated with the antitumour agents flavone-8-acetic acid and xanthenone-4-acetic acid. Cancer Res., *50*: 6966-6970, 1990.
22) Ching, L.M., and Baguley, B.C. Enhancement of in vitro cytotoxicity of mouse peritoneal exudate cells by flavone acetic acid (NSC 347512). Eur. J. Cancer Clin. Oncol, *24*: 1521-1525, 1988.
23) Mahadevan, V., Malik, S.T., Meager, A., Fiers, W., Lewis, G.P., and Hart, I.R. Role of tumour necrosis factor in flavone acetic acid-induced tumour vasculature shutdown. Cancer Res., *50:* 5537-5542, 1990.
24) Ching, L.M., Joseph, W.R., Crosier, K.E., and Baguley, B.C. Induction of tumour necrosis factor-alpha messenger RNA in human and murine cells by the flavone acetic acid analogue 5,6-dimethylxanthenone-4-acetic acid (NSC 640488). Cancer Res., *54*: 870-872, 1994.
25) Ching, L.M., Goldsmith, D., Joseph, W.R., Körner, H., Sedgwick, J.D., and Baguley, B.C. Induction of intratumoral tumour necrosis factor (TNF) synthesis and hemorrhagic necrosis by 5,6-Dimethylxanthenone-4-acetic acid (DMXAA) in TNF knockout mice. Cancer Res., *59*:3304-3307, 1999.
26) Ruegg, C. Yilmaz, A., Bieler, G., Bamat, J., Chaubert, P., and Lejeune, F.J. Evidence for the involvement of endothelial cell integrin alphaVbeta3 in the disruption of the tumour vasculature induced by TNF and IFN-gamma. Nature Med., *4*: 408-414, 1998.
27) Ching, L-M., Joseph, W.R-, and Baguley, B.C. Antitumour responses to flavone-8-acetic and 5,6-dimethylxanthenone-4-acetic acid in immune deficient mice. Br. J. Cancer, *66*: 128-130, 1992.
28) Bibby, M.C., Phillips, R.M., Double, J.A., and Pratesi, G. Anti-tumour activity of flavone acetic acid (NSC 347512) in mice-influence of immune status. Br. J. Cancer, *63*: 57-62, 1991.
29) Pratesi, G., Rodolfo, M., Rovetta, G., and Parmiani, G. Role of T cells and tumour necrosis factor in antitumour activity and toxicity of flavone acetic acid. Eur. J. Cancer, *26*: 1079-1083, 1990.
30) Melcher, A., Todryk, S., Hardwick, N., Ford, M., Jacobson, M., and Vile, R.G. Tumour immunogenicity is determined by the mechanism of cell death via induction of heat shock protein expression. Nature Med., *4*: 581-586, 1998.
31) Todryk, S., Melcher, A.A, Hardwick, N., Linardakis, .E, Bateman, A., Colombo, M.P., Stoppacciaro, A., and Vile, R.G. Heat shock protein 70 induced during tumour cell killing induces Th1 cyto1rinf!$ and targets immature dendritic cen precursors to enhance antigen uptake. J. Immunol, *163*: 1398-1408, 1999.
32) Azuma, M., Cayabyab, M., Buck, D., Phillips, J.H., and Lanier, L.L. Involvement of CD28 in MHC-unrestricted cytotoxicity mediated by a human natural killer leukemia cell lime. J. Immunol., *149*: 1115-1123, 1992.
33) Ryan, H.E., Lo, J., Johnson, R.S. HIF-1α is required for solid tumour formation and embryonic vascularization. *EMBO J*. 1998; *17*: 3005-3015.

## Claims

1. Use of an immunotherapeutic agent in conjunction with a tumour growth restricting agent, for the manufacture of a medicament for the treatment of mammals with advanced or large tumour burdens, wherein the tumour growth restricting agent potentiates the activity of the immunotherapeutic agent and is one of 5,6-dimethylxanthenone-4-acetic acid (DXMAA) and flavone acetic acid (FAA) and wherein the immunotherapeutic agent comprises a T-cell co-stimulatory cell adhesion molecule (CAM) or a mammalian expression vector containing DNA which encodes a T-cell co-stimulatory CAM, wherein the CAM is B7.1.

2. The use according to claim 1 wherein the tumour growth restricting agent is 5,6-dimethylxanthenone-4-acetic acid (DXMAA).

3. The use according to claim 1 wherein the tumour growth restricting agent is flavone acetic acid (FAA).

4. The use according to any preceding claim wherein the mammal is a human.

5. Use as claimed in any preceding claim, wherein the medicament is formulated for administration of the immunotherapeutic agent prior to the administration of the tumour growth restricting agent.

6. Use as claimed in claim 5, wherein the medicament is formulated for administration of the immunotherapeutic agent from 12 to 24 hours prior to the administration of the tumour growth restricting agent.

7. Use as claimed in any preceding claim, wherein the medicament further includes an additional tumour growth restricting agent.

## Patentansprüche

1. Verwendung eines immuntherapeutischen Mittels zusammen mit einem Tumorwachstum begrenzenden Mittel zur Herstellung eines Medikaments für die Behandlung von Säugern mit fortgeschrittener oder großer Tumorbelastung, wobei das Tumorwachstum begrenzende Mittel die Wirksamkeit des immuntherapeutischen Mittels verstärkt und eines von 5,6-Dimethylxanthenon-4-essigsäure (DXMAA) und Flavonessigsäure (FAA) ist und wobei das immuntherapeutische Mittel ein T-Zellen kostimulierendes Zelladhäsionsmolekül (CAM) oder einen Säugerexpressionsvektor, der DNA enthält, welche ein T-Zellen kostimulierendes CAM codiert, umfaßt, wobei das CAM B7.1 ist.

2. Verwendung nach Anspruch 1, wobei das Tumorwachstum begrenzende Mittel 5,6-Dimethylxanthenon-4-essigsäure (DXMAA) ist.

3. Verwendung nach Anspruch 1, wobei das Tumorwachstum begrenzende Mittel Flavonessigsäure (FAA) ist.

4. Verwendung nach einem der vorangegangenen Ansprüche, wobei der Säuger ein Mensch ist.

5. Verwendung nach einem der vorangegangenen Ansprüche, wobei das Medikament für die Verabreichung des immuntherapeutischen Mittels vor der Verabreichung des Tumorwachstum begrenzenden Mittels formuliert ist.

6. Verwendung nach Anspruch 5, wobei das Medikament für die Verabreichung des immuntherapeutischen Mittels 12 bis 24 Stunden vor der Verabreichung des Tumorwachstum begrenzenden Mittels formuliert ist.

7. Verwendung nach einem der vorangegangenen Ansprüche, wobei das Medikament weiterhin ein zusätzliches Tumorwachstum begrenzendes Mittel enthält.

## Revendications

1. Utilisation d'un agent immunothérapeutique conjointement avec un agent de restriction de croissance tumorale, pour la production d'un médicament destiné au traitement de mammifères présentant des charges tumorales avancées ou importantes, dans laquelle l'agent de restriction de croissance tumorale potentialise l'activité de l'agent immunothérapeutique et est un des acides consistant en l'acide 5,6-dimêthylxanthénone-4-acétique (DXMAA) et l'acide flavone-acétique (FAA), et dans laquelle l'agent immunothérapeutique comprend une molécule d'adhésion cellulaire (CAM) costimulatrice des lymphocytes T ou un vecteur d'expression d'un mammifère contenant un ADN qui code pour une CAM costimulatrice des lymphocytes T. la CAM étant la CAM B7.1.

2. Utilisation suivant la revendication 1, dans laquelle l'agent de restriction de croissance tumorale est l'acide 5,6-diméthylxanthénone-4-acétique (DXMAA).

3. Utilisation suivant la revendication 1, dans laquelle l'agent de restriction de croissance tumorale est l'acide flavone-acétique (FAA).

4. Utilisation suivant l'une quelconque des revendications précédentes, dans laquelle le mammifère est un être humain.

5. Utilisation suivant l'une quelconque des revendications précédentes, dans laquelle le médicament est formulé pour l'administration de l'agent immunothérapeutique avant l'administration de l'agent de restriction de croissance tumorale.

6. Utilisation suivant la revendication 5, dans laquelle le médicament est formulé pour l'administration de l'agent immunothérapeutique 12 à 24 heures avant l'administration de l'agent de restriction de croissance tumorale.

7. Utilisation suivant l'une quelconque des revendications précédentes, dans laquelle le médicament comprend en outre un agent de restriction de croissance tumorale supplémentaire.
